# EUROPÄISCHE PATENTANMELDUNG

(11) **EP 1 859 777 A2**
(43) Veröffentlichungstag der Anmeldung: **28.11.2007**
(21) Anmeldenummer: 07009018.8
(22) Anmeldetag: 04.05.2007
(51) Int. Cl.: A61K 8/36, A61Q 9/02

(54) **Elektrolythaltige Seifenzusammensetzungen**

(30) Priorität: 23.05.2006 DE 102006024517
(71) Anmelder: Henkel Kommanditgesellschaft auf Aktien, 40589 Düsseldorf (DE)
(72) Erfinder: Pujol Galiano, Miracle, 08329 Teià (ES); Lapena, Margarita, 08026 Barcelona (ES)

(57) **Zusammenfassung**

Gegenstand der vorliegenden Erfindung sind schäumbare kosmetische Zusammensetzungen, insbesondere Rasiercremes, mit verbesserter Herstellbarkeit auf der Basis wasser- und elektrolythaltiger Seifenzusammensetzungen, die bei Temperaturen von 20 bis 90°C leicht verformbar und zur Abgabe aus einer Tube oder aus einem Dosierspender geeignet sind.

## Beschreibung

Die vorliegende Erfindung betrifft schäumbare kosmetische Zusammensetzungen, insbesondere Rasiercremes, mit verbesserter Herstellbarkeit auf der Basis wasser- und elektrolythaltiger Seifenzusammensetzungen, die bei Temperaturen von 20 bis 90°C leicht verformbar und zur Abgabe aus einer Tube oder aus einem Dosierspender geeignet sind.
Als Rasiercreme im Sinne der vorliegenden Erfindung ist ein Hilfsmittel für die manuelle Nassrasur unter Verwendung einer Rasierklinge zu verstehen, das in Gegenwart von Wasser und unter mechanischer Einarbeitung von Luft mit Hilfe eines Pinsels oder Schwammes in einer Seifenschale oder direkt auf der Haut einen feinblasigen, stabilen Schaum entwickelt. Dieser Schaum soll vor der Rasur die behaarte Haut und die zu entfernenden Haare benetzen und gleitfähig machen und das Barthaar weich machen.
Darüber hinaus muss ein solches Produkt gute Benetzungseigenschaften aufweisen und darf die Haut, insbesondere vor einer die Haut sowieso beanspruchende Rasur, nicht reizen.
Für die dosierte Abgabe aus einer Tube, insbesondere einer Quetschtube, oder aus einem Pumpspender, ist eine cremeförmige, fließfähige Konsistenz notwendig.
Rasiercremes auf der Basis von Fettsäureseifen sind im Stand der Technik gut bekannt, da Seife die Anforderungen an einen feinblasigen Schaum und an eine gel- oder cremeförmige Konsistenz gut erfüllt und darüber hinaus ein preiswerter Rohstoff ist. Auch wirkt sich der alkalische pH der Seifen günstig auf die Erweichung des Barthaars aus und bereitet dieses somit optimal auf die Rasur vor.
Herkömmliche wasserhaltige Rasiercremes auf Basis von Alkaliseifen weisen in Bezug auf die Herstellbarkeit sowie auf die Lagerung oberhalb Normaltemperatur (20°C) erhebliche Probleme auf. Zur Herstellung derartiger Rasiercremes wird üblicherweise eine Mischung von Natron- und Kalilauge zu der aufgeschmolzenen Fettsäure gegeben. Die ablaufende Neutralisierung wird als Verseifung, die entstehenden Fettsäuresalze als Seifen bezeichnet. Die *in situ* entstehenden Fettsäurealkalisalze bilden ein Gel, was mit einem erheblichen Anstieg der Viskosität des Systems verbunden ist. Die Überwindung der hohen Scherkräfte in derartig verfestigten Mischungen ist nicht mehr mit konventionellen Rührern möglich, sondern bedarf aufwendiger apparativer Maßnahmen. Aber selbst mit aufwendigen Mischapparaturen erzielt man nur Produkte unzureichender Homogenität und geringer optischer Attraktivität.
Ein weiteres Problem ist die Lagerstabilität derartiger Zusammensetzungen, insbesondere bei höheren Temperaturen (30 - 40 °C und darüber). Werden konzentrierte Seifenzubereitungen eine Zeit lang bei höheren Temperaturen gelagert, verlieren sie ihre anfängliche cremeartige, fließfähige Konsistenz und verfestigen sich irreversibel. Für kosmetische Produkte können höhere Lagertemperaturen beispielsweise in warmen Ländern, im Sommer oder in schlecht klimatisierten Transportfahrzeugen, Warenlagern und Ladenlokalen auftreten. Eine Verfestigung der Zusammensetzung, die sich auch bei anschließender längerer Lagerung bei Raumtemperatur nicht ausreichend zurückbildet, erschwert die Entnahme aus der Verpackung oder macht sie sogar unmöglich, wodurch das Produkt unbrauchbar und für den Verbraucher inakzeptabel wird. Auch der (relativ geringe) Perlglanz der Seifenzubereitung geht irreversibel verloren.
Bekannte Rasiercremes überwinden diese Nachteile durch einen Zusatz an Dinatriumtetraborat (Borax). Borax senkt den Viskositätsanstieg konzentrierter Seifenzubereitungen bei höheren Temperaturen, wodurch die Herstellung erleichtert und die Lagerstabilität verbessert werden. Außerdem bilden die Borax-haltigen Seifenzubereitungen nach der Herstellung und Reifung bei Raumtemperatur einen schönen Perlglanz aus, der die Ästhetik und Attraktivität des Produktes erheblich erhöht. Weiterhin beschleunigt Borax in vorteilhafter Weise den Reifungsprozess der Seifenzubereitungen, das heißt, bereits 2 bis 3 Tage nach Herstellung und Lagerung bei Raumtemperatur entwickelt sich der schöne Perlglanz und bereits 4 bis 5 Tage nach Herstellung und Lagerung bei Raumtemperatur hat sich die Viskosität stabilisiert. Aufgrund verschärfter gesetzlicher Regulierungen sollte der Zusatz von Borax zu kosmetischen Produkten möglichst vermieden werden. Das Weglassen von Borax ohne die Zugabe eines adäquaten Ersatzstoffes führt aber zum Auftreten der oben genannten Probleme.

Für den Fachmann stellte sich daher die Aufgabe, einen zugelassenen Ersatzstoff für Borax zu finden, der eine vergleichbar einfache Herstellung und Handhabung konzentrierter Seifenzubereitungen mit cremiger, pastöser Konsistenz ermöglicht und ebenfalls die Ausbildung eines attraktiven Perlglanzes der Zusammensetzungen begünstigt.
Eine weitere Aufgabe war es, einen zugelassenen Ersatzstoff für Borax zu finden, der eine vergleichbar schnelle Einstellung der Endviskosität und/oder des Perlglanzes ("Reifung") konzentrierter Seifenzubereitungen mit cremiger, pastöser Konsistenz ermöglicht.
Eine weitere Aufgabe war es, einen zugelassenen Ersatzstoff für Borax zu finden, der eine vergleichbar einfache Herstellung und Handhabung konzentrierter Seifenzubereitungen mit cremiger, pastöser Konsistenz und hoher Lagerstabilität, vor allem bei höheren Temperaturen, ermöglicht.
Eine weitere Aufgabe war es, kosmetische Cremes auf der Basis konzentrierter Seifenzubereitungen mit guter Hautverträglichkeit und geringem Irritationspotenzial bereitzustellen, die sich insbesondere als Rasiercremes, aber auch zur Haut- und Haarreinigung eignen.

Im Stand der Technik ist bekannt, dass die oben dargelegten Effekte von Borax auf konzentrierte Seifenzubereitungen auf der Elektrolytwirkung von Borax beruhen. Allerdings wurde festgestellt, dass nicht alle Elektrolyten dieselbe Wirkung wie Borax haben. Einige Elektrolyten führten zu einer zu starken Reduzierung der Viskosität, so dass sich keine cremige, pastöse Konsistenz des Endproduktes einstellte. Andere Elektrolyten bildeten klumpige, inhomogene Zusammensetzungen und waren somit ebenfalls ungeeignet.

Überraschend wurde gefunden, dass ausgewählte Elektrolyten einen adäquaten Ersatz für Borax bei der Herstellung lagerstabiler, perlglänzender konzentrierter Seifenzubereitungen darstellen.

Gegenstand der vorliegenden Anmeldung sind perlglänzende kosmetische Zusammensetzungen, die als Rasiercreme verwendet werden können, enthaltend Wasser, mindestens eine Monocarbonsäure mit 8 - 24 Kohlenstoffatomen, ausgewählt aus linearen, verzweigten, gesättigten und ungesättigten Monocarbonsäuren mit 8 - 24 Kohlenstoffatomen, mindestens ein Neutralisierungsmittel für die Monocarbonsäure/n, sowie mindestens einen Elektrolyten, der ausgewählt ist aus Natriumchlorid, Natriumsulfat, Natriumlactat, Natriumglycolat, Natriumcitrat, Kaliumchlorid, Kaliumsulfat, Magnesiumchlorid, Calciumchlorid sowie Mischungen hiervon, in einer Gesamtmenge von 0,5 -10 Gew.-%, bezogen auf das Gewicht der gesamten Zusammensetzung.

Erfindungsgemäß bevorzugte C₈ - C₂₄-Monocarbonsäuren sind ausgewählt aus linearen und verzweigten, gesättigten und ungesättigten Monocarbonsäuren, die mit funktionellen Gruppen, beispielsweise OH-Gruppen, substituiert sein können. Bevorzugt sind Octansäure, Nonansäure, Decansäure, Decensäure, Undecansäure, Undecensäure, Dodecansäure (Laurinsäure), Dodecensäure, Tridecansäure, Tridecensäure, Myristinsäure, Tetradecensäure, lsotetradecansäure, Pentadecansäure, Pentadecensäure, Palmitinsäure, Hexadecensäure, Palmitoleinsäure, Isohexadecansäure, 2-Hexyldecansäure (Isopalmitinsäure) Stearinsäure, Ölsäure, Linolsäure, Linolensäure, Isostearinsäure, 2-Octyldecansäure, Ricinolsäure, hydrierte Ricinolsäure (= 12-Hydroxystearinsäure), Eicosansäure, Eicosensäure, Arachidonsäure und Behensäure. Erfindungsgemäß besonders bevorzugte C₈ - C₂₄-Monocarbonsäuren sind ausgewählt aus Decansäure, Undecansäure, Laurinsäure, Tridecansäure, Myristinsäure, Palmitinsäure, lsopalmitinsäure, Stearinsäure, Isostearinsäure, Ölsäure, Linolsäure, Eicosansäure und Behensäure. Außerordentlich bevorzugt sind Palmitinsäure, Stearinsäure, Laurinsäure, Ölsäure und Linolsäure sowie Mischungen hiervon. Weiterhin außerordentlich bevorzugt sind Mischungen aus Palmitinsäure und Stearinsäure sowie Mischungen aus Palmitinsäure, Stearinsäure und Laurinsäure.
Bevorzugte erfindungsgemäße Zusammensetzungen sind dadurch gekennzeichnet, dass sie mindestens eine Monocarbonsäure mit 8 - 24 Kohlenstoffatomen in einer Gesamtmenge von 25 - 50 Gew.-%, bevorzugt 30 - 45 Gew.-% und besonders bevorzugt 38 - 42 Gew.-%, enthalten, jeweils bezogen auf das Gesamtgewicht der Zusammensetzung. Zur Berechnung des Gewichtsanteils der C₈ - C₂₄-Monocarbonsäuren wird das Gewicht in Bezug auf die nichtneutralisierte Säure verwendet, unabhängig vom Vorliegen als Salz (Seife) oder freie Säure und unabhängig von der Art des Gegenions der neutralisierten (Salz-)Form (Seife).

Die mindestens eine C₈ - C₂₄-Monocarbonsäure liegt in den erfindungsgemäßen Zusammensetzungen ganz oder teilweise in mit üblichen alkalischen Substanzen (wie insbesondere bevorzugt Natrium- und/oder Kaliumhydroxid, Natrium- und/oder Kaliumcarbonat sowie Mono- und/oder Triethanolamin) neutralisierter Form vor und wird in dieser Form auch als Seife bezeichnet. Bevorzugte Seifen sind Natriumstearat, Kaliumstearat, Triethanolaminstearat, Natriumisopalmitat, Kaliumisopalmitat, Triethanolaminisopalmitat, Natriumpalmitat, Kaliumpalmitat, Triethanolaminpalmitat, Natriumbehenat, Kaliumbehenat, Triethanolaminbehenat, Natriummyristat, Kaliummyristat, Triethanolaminmyristat, Natriumlaurat, Kaliumlaurat, Triethanolaminlaurat, Natriumoleat, Kaliumoleat und Triethanolaminoleat sowie Mischungen hiervon.
Bevorzugte erfindungsgemäße Zusammensetzungen sind dadurch gekennzeichnet, dass die Gesamtmenge an nicht-neutralisierter Monocarbonsäure/nicht-neutralisierten Monocarbonsäuren mit 8 - 24 Kohlenstoffatomen b) 0,5 - 5 Gew.-%, bevorzugt 1 - 4 Gew.-%, jeweils bezogen auf das Gewicht der gesamten Zusammensetzung, beträgt.

Weitere bevorzugte erfindungsgemäße Zusammensetzungen sind dadurch gekennzeichnet, dass die Gesamtmenge an ungesättigter Monocarbonsäure/ungesättigten Monocarbonsäuren b) mit 8 - 24 Kohlenstoffatomen 0,01 - 20 Gew.-%, bevorzugt 0,3 - 2 Gew.-%, jeweils bezogen auf das Gewicht der gesamten Zusammensetzung, beträgt. Besonders bevorzugte ungesättigte Monocarbonsäuren b) mit 8 - 24 Kohlenstoffatomen sind ausgewählt aus Ölsäure und Linolsäure.

Weitere bevorzugte erfindungsgemäße Zusammensetzungen sind dadurch gekennzeichnet, dass der Anteil an Laurinsäure 2 - 10 Gew.-%, bevorzugt 3 - 6 Gew.-%, jeweils bezogen auf das Gewicht der gesamten Zusammensetzung, beträgt.

Weitere bevorzugte erfindungsgemäße Zusammensetzungen sind dadurch gekennzeichnet, dass die Gesamtmenge an Stearinsäure und/oder Palmitinsäure 20 - 40 Gew.%, bevorzugt 25 - 35 Gew.-%, jeweils bezogen auf das Gewicht der gesamten Zusammensetzung, beträgt.
Besonders bevorzugt sind Mischungen von Stearat und Palmitat, insbesondere solche Mischungen mit einem Gewichtsverhältnis von Palmitat zu Stearat von 1,5:1 bis 3:1, bevorzugt 2:1 bis 7:3. Ein bevorzugtes entsprechendes Handelsprodukt, das im wesentlichen Palmitin- und Stearinsäure enthält, ist z. B. Edenor® L2 SM, das von der Firma Cognis kommerziell vertrieben wird.
Als verzweigte Monocarbonsäuren mit 8 - 24 Kohlenstoffatomen kommen bevorzugt 2-Hexyldecansäure und 2-Octyldecansäure in Frage. Besonders bevorzugt ist 2-Hexyldecansäure, auch als Isopalmitinsäure bezeichnet, die von der Firma Cognis unter der Bezeichnung Edenor IP 95 vertrieben wird. Verzweigte Monocarbonsäuren mit 8 - 24 Kohlenstoffatomen sind in den erfindungsgemäßen Zusammensetzungen bevorzugt in einer Gesamtmenge von 8 bis 18 Gew.-%, besonders bevorzugt in einer Menge von 14 bis 16 Gew.-%, jeweils bezogen auf das Gewicht der gesamten Zusammensetzung, enthalten.

Weitere bevorzugte erfindungsgemäße Zusammensetzungen sind dadurch gekennzeichnet, dass die mindestens eine Monocarbonsäure b) mit 8 - 24 Kohlenstoffatomen in situ gebildet wird aus mindestens einem Glyceryltriester entsprechender Monocarbonsäuren, der bei 30 °C flüssig ist, und dem Neutralisierungsmittel für b). Das bedeutet, dass den Fettsäuren vor der Verseifung mindestens ein Glyceryltriester von Monocarbonsäuren mit 8 - 24 Kohlenstoffatomen, der bei 30 °C flüssig ist, zugesetzt wird, der durch das/die Neutralisierungsmittel c) in die entsprechenden Monocarbonsäuren und Glycerin gespalten wird. In einer besonders bevorzugten Ausführungsform der Erfindung ist der mindestens eine Glyceryltriester ausgewählt aus natürlichen Ölen, vorzugsweise aus Avocadoöl, Baumwollsaatöl, Distelöl, Erdnussöl, Kokosöl, Leinöl, Maiskeimöl, Mandelöl, Olivenöl, Palmöl, Palmkernöl, Pfirsichkernöl, Rapsöl, Rizinusöl, Sesamöl, Sojaöl, Sonnenblumenöl und Weizenkeimöl.
Weitere bevorzugte erfindungsgemäße Zusammensetzungen sind dadurch gekennzeichnet, dass das Neutralisierungsmittel c) ausgewählt ist aus Kaliumhydroxid, Triethanolamin, Mischungen von Kaliumhydroxid und Natriumhydroxid, Mischungen von Triethanolamin und Natriumhydroxid und Mischungen von Kaliumhydroxid, Triethanolamin und Natriumhydroxid. Bevorzugt sind Mischungen von Kaliumhydroxid und Natriumhydroxid. Besonders bevorzugt sind Mischungen, in denen Kaliumhydroxid im molaren Überschuss zu Natriumhydroxid vorliegt, Mischungen, in denen Triethanolamin im molaren Überschuss zu Natriumhydroxid vorliegt und Mischungen, in denen die Gesamtmenge an Kaliumhydroxid und Triethanolamin im molaren Überschuss zu Natriumhydroxid vorliegt.

Weitere bevorzugte erfindungsgemäße Zusammensetzungen sind dadurch gekennzeichnet, dass die Gesamtmenge an Wasser 30 - 60 Gew.-%, bevorzugt 35 - 50 Gew.-%, besonders bevorzugt 40 - 45 Gew.-%, jeweils bezogen auf das Gewicht der gesamten Zusammensetzung, beträgt.

Weitere bevorzugte erfindungsgemäße Zusammensetzungen sind dadurch gekennzeichnet, dass der mindestens eine Elektrolyt d), der ausgewählt ist aus Natriumchlorid, Natriumsulfat, Natriumlactat, Natriumglycolat, Natriumcitrat, Kaliumchlorid, Kaliumsulfat, Magnesiumchlorid, Calciumchlorid sowie Mischungen hiervon, in einer Gesamtmenge von 0,6 - 5 Gew.-%, bevorzugt 0,9 - 2,0 Gew.-%, außerordentlich bevorzugt 1,2 - 1,8 Gew.-%, jeweils bezogen auf das Gewicht der gesamten Zusammensetzung, vorliegt. Besonders bevorzugte erfindungsgemäße Zusammensetzungen sind durch einen Gehalt an Natriumlactat gekennzeichnet, insbesondere durch einen Natriumlactatgehalt von 0,6 - 5 Gew.-%, außerordentlich bevorzugt durch einen Natriumlactatgehalt von 0,9 - 2,0 Gew.-%.

Die vorgenannten Elektrolyten d) dienen als Ersatz für Borax (= Dinatriumtetraborat). Besonders bevorzugte erfindungsgemäße Zusammensetzungen sind deshalb dadurch gekennzeichnet, dass weder Borsäure noch ein Salz der Borsäure noch ein Derivat der Borsäure enthalten ist.

Mit den vorgenannten Elektrolyten bilden die konzentrierten Seifenzubereitungen nach der Herstellung und Reifung bei Raumtemperatur einen schönen Perlglanz aus, der die Ästhetik und Attraktivität des Produktes erheblich erhöht. Weiterhin beschleunigen die vorgenannten Elektrolyte in vorteilhafter Weise den Reifungsprozess der Seifenzubereitungen, das heißt, bereits 2 bis 3 Tage nach Herstellung und Lagerung bei Raumtemperatur entwickelt sich ein attraktiver Perlglanz und bereits 4 bis 5 Tage nach Herstellung und Lagerung bei Raumtemperatur stabilisiert sich die Viskosität und erreicht nahezu ihren Endwert. Das bedeutet, dass die erfindungsgemäßen Zusammensetzungen auch ohne typische Perlglanzgeber, wie Ethylenglycolmonostearat, Ethylenglycoldistearat, Diethylenglycolmonostearat, Diethylenglycoldistearat oder Fettsäuremonoethanolamide, einen ausgeprägten Perlglanz entwickeln.
Besonders bevorzugte erfindungsgemäße Zusammensetzungen sind daher dadurch gekennzeichnet, dass maximal 0,2 Gew.-%, bevorzugt maximal 0,1 Gew.-%, besonders bevorzugt 0 Gew.-%, jeweils bezogen auf das Gewicht der gesamten Zusammensetzung, Ethylenglycolmono- und/oder -distearat und/oder Diethylenglycolmono- und/oder -distearat und/oder Fettsäuremonoethanolamid enthalten ist/sind.

Die erfindungsgemäßen Seifenzubereitungen, die als Rasiercremes verwendet werden, verbleiben typischerweise eine gewisse Zeit auf der Haut, da sie beim Rasieren nach und nach abgetragen und entfernt werden. Zumindest ein Teil der Zusammensetzung trocknet dabei etwas an, was ein unangenehmes Spannungsgefühl auf der Haut verursachen kann. Weitere bevorzugte erfindungsgemäße Zusammensetzungen enthalten daher mindestens ein Feuchthaltemittel, ausgewählt aus wasserlöslichen mehrwertigen C₂ - C₉-Alkanolen mit 2 - 6 Hydroxylgruppen und wasserlöslichen Polyethylenglycolen, bestehend aus 3 - 20 Ethylenoxid-Einheiten. Unter Wasserlöslichkeit versteht man erfindungsgemäß, dass sich wenigstens 5 Gew.-% des Polyols bei 20 °C in Wasser klar lösen oder aber - im Falle längerkettiger Polyole - durch Erwärmen der Lösung auf 50 °C bis 60 °C in Lösung gebracht werden können.
Außerdem wurde überraschend festgestellt, dass durch den Zusatz von mindestens einem Feuchthaltemittel, wie vorstehend aufgeführt, Verbesserungen bei der Herstellung, wie verkürzte Zeit bis zur Einstellung des Perlglanzes und/oder bis zur Stabilisierung der Viskosität, erzielt werden konnten.
Besonders bevorzugte wasserlösliche mehrwertige C₂ - C₉-Alkanole mit 2 - 6 Hydroxylgruppen sind ausgewählt aus 1,2-Propylenglycol, 1,3-Propylenglycol, 2-Methyl-1,3-propandiol, Glycerin, 1,2-Butylenglycol, 1,3-Butylenglycol und 1,4-Butylenglycol, Pentylenglycolen wie 1,2-Pentandiol und 1,5-Pentandiol, Hexandiolen wie 1,6-Hexandiol und 1,2-Hexandiol, Hexantriolen wie 1,2,6-Hexantriol, 1,2-Octandiol, 1,8-Octandiol, Dipropylenglycol, Tripropylenglycol, Diglycerin, Triglycerin, Erythrit, Pentaerythrit, Trimethylolpropan, Sorbit, Monoanhydrosorbiten (Sorbitanen), Cyclohexantriol, Inositen, Fructose, Glucose, Maltose, Maltitol, Mannit, Sucrose, Trehalose, Xylose sowie Mischungen der vorgenannten Substanzen.

Besonders bevorzugte wasserlösliche Polyethylenglycole, bestehend aus 3 - 20 Ethylenoxid-Einheiten, sind ausgewählt aus PEG-3, PEG-4, PEG-6, PEG-7, PEG-8, PEG-9, PEG-10, PEG-12, PEG-14, PEG-16, PEG-18 und PEG-20 sowie Mischungen hiervon, wobei PEG-3 bis PEG-8 bevorzugt sind.
Weitere besonders bevorzugte erfindungsgemäße Zusammensetzungen sind dadurch gekennzeichnet, dass mindestens ein wasserlösliches mehrwertiges C₂ - C₉-Alkanol mit 2 - 6 Hydroxylgruppen und/oder mindestens ein wasserlösliches Polyethylenglycol, bestehend aus 3 - 20 Ethylenoxid-Einheiten, sowie Mischungen hiervon, in einer Gesamtmenge von 2 - 20 Gew.-%, bevorzugt 5 - 15 Gew.-%, besonders bevorzugt 7 - 12 Gew.-%, jeweils bezogen auf das Gewicht der gesamten Zusammensetzung, enthalten ist/sind.

Die erfindungsgemäßen Seifenzubereitungen weisen bereits ein gutes Schaumvermögen auf. Um den hohen Ansprüchen der Verbraucher an ein besonders schnelles Anschäumen gerecht zu werden, bestand weiterhin die Aufgabe, eine konzentrierte Seifenzubereitung bereitzustellen, die sich nicht nur einfach herstellen lässt und eine hohe Lagerstabilität und einen attraktiven Perlglanz aufweist, sondern die auch ein besonders schnelles Anschäumverhalten und/oder eine hohe Schaumstabilität zeigt. Es wurde gefunden, dass durch die Zugabe eines zusätzlichen anionischen Tensids, das nicht der Klasse der ebenfalls anionischen Fettsäuresalze (Seifen) zuzuordnen ist, das Anschäumverhalten der erfindungsgemäßen Zusammensetzungen weiter optimiert werden konnte. Unter Optimierung des Anschäumverhaltens wird erfindungsgemäß verstanden, dass innerhalb der ersten Sekunden, in denen die Seifenzubereitung angerührt wird, eine starke Schaumbildung einsetzt und dieser "Flash foam" sein Volumen auch für einige Zeit hält und nicht sofort wieder zusammenfällt. Insbesondere war überraschend, dass hierzu schon relativ geringe Mengen des zusätzlichen Aniontensids ausreichen. Weitere bevorzugte erfindungsgemäße Zusammensetzungen sind daher dadurch gekennzeichnet, dass mindestens ein weiteres anionisches Tensid enthalten ist.

Als mit Bezug auf die Seifen zusätzliche anionische Tenside eignen sich in den erfindungsgemäßen Zusammensetzungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit 8 bis 30 C-Atomen. Zusätzlich können im Molekül Glycol- oder Polyglycolether-Gruppen, Ester-, Ether- und Amidgruppen sowie Hydroxylgruppen enthalten sein. Beispiele für bevorzugte anionische Tenside sind, jeweils in Form ihrer Salze:
- Ethercarbonsäuren der Formel R-O-(CH₂-CH₂O)ₓ-CH₂-COOH, in der R eine lineare Alkylgruppe mit 8 - 30, bevorzugt 8 - 18 C-Atomen, und x = 0 oder 1 - 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und -dialkylester mit 8 - 24, bevorzugt 8 - 18 C-Atomen in der Alkylgruppe und Sulfobernsteinsäuremono-alkylpolyoxyethylester mit 8 - 24, bevorzugt 8 - 18 C-Atomen in der Alkylgruppe und 1 bis 6 Glycolethergruppen im Molekül,
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- Alkylsulfate mit 8 -18 C-Atomen in der Alkylgruppe,
- Alkylpolyglycolethersulfate der Formel R-O(CH₂-CH₂O)ₓOSO₃H, in der R eine bevorzugt lineare Alkylgruppe mit 8 - 30 C-Atomen, bevorzugt 8 - 18 C-Atomen, und x = 0 oder 1 - 12, bevorzugt 1 - 4, ist,
- Ester der Weinsäure und Zitronensäure mit Alkoholen, die Anlagerungsprodukte von etwa 2-15 Ethylenoxid und/oder Propylenoxid-Einheiten an Fettalkohole mit 8 bis 22 C-Atomen darstellen, insbesondere Citronensäurealkylpolyglycolethermonoester, Citronensäurealkylpolyglycoletherdiester, Weinsäurealkylpolyglycolethermonoester und Weinsäurealkylpolyglycoletherdiester, besonders bevorzugt solche, bei denen der Alkylrest von linearen Fettalkoholen mit 8 bis 18, bevorzugt 12 C-Atomen, stammt und durchschnittlich 5 -10, bevorzugt durchschnittlich 7 - 9, Ethylenoxid-Einheiten pro Molekül enthalten sind,
- Alkyl- und/oder Alkenyletherphosphate der Formel (II), 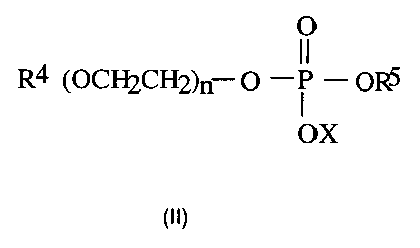 in der R⁴ bevorzugt für einen aliphatischen Kohlenwasserstoffrest mit 8 - 30, bevorzugt 8 - 18 C-Atomen, R⁵ für Wasserstoff, einen Rest (CH₂CH₂O)ₙR¹⁸ oder X, n für Zahlen von 1 bis 10 und X für Wasserstoff, ein Alkali- oder Erdalkalimetall oder NR⁶R⁷R⁸R⁹, mit R⁶ bis R⁹ unabhängig voneinander stehend für Wasserstoff oder einen C₁ - C₄ - Kohlenwasserstoffrest, steht,
- sulfatierte Fettsäurealkylenglycolester der Formel R¹⁰CO(AlkO)ₙSO₃M, in der R¹⁰CO- für einen linearen oder verzweigten, aliphatischen, gesättigten und/oder ungesättigten Acylrest mit 6 bis 22 C-Atomen, Alk für CH₂CH₂, CHCH₃CH₂ und/oder CH₂CHCH₃, n für Zahlen von 0,5 bis 5 und M für ein Kation steht, wie sie in der DE-OS 197 36 906.5 beschrieben sind,
- Monoglyceridsulfate und Monoglyceridethersulfate der Formel (IV) 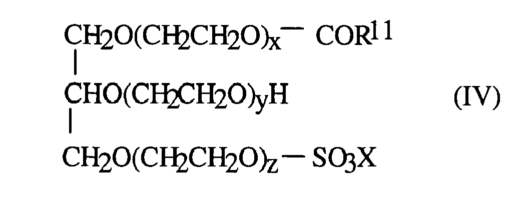 in der R¹¹CO für einen linearen oder verzweigten Acylrest mit 6 - 22, bevorzugt 8 - 18 C-Atomen, x, y und z in Summe für 0 oder für Zahlen von 1 bis 30, vorzugsweise 2 bis 10, und X für ein Alkali- oder Erdalkalimetall steht,
- Amidethercarbonsäuren gemäß EP 0 690 044,
- Kondensationsprodukte aus C₈ - C₃₀ - Fettalkoholen, bevorzugt C₈ - C₁₈ - Fettalkoholen mit Proteinhydrolysaten und/ oder Aminosäuren und deren Derivaten, sogenannte Eiweißfettsäurekondensate, z. B. Lamepon^{®}, Gluadin^{®}, Hostapon^{®} KCG oder Amisoft^{®}.

Die Salze dieser Tenside sind bevorzugt ausgewählt aus den Natrium-, Kalium- und Ammoniumsowie den Mono-, Di- und Trialkanolammoniumsalzen mit 2 bis 4 C-Atomen in der Alkanolgruppe. In der Regel sind die Natriumsalze der genannten Aniontenside bei 20°C fest, wohingegen die Mono-, Di- und Trialkanolammoniumsalze mit 2 bis 4 C-Atomen in der Alkanolgruppe in der Regel bei 20°C flüssig oder in pastöser, fließfähiger Form vorliegen. Ein bevorzugtes Tensid dieser Art ist ein Tensidgemisch von Alkylpolyglycolethersulfaten der Formel R-O(CH₂-CH₂O)ₓ-OSO₃H, in der R eine lineare Alkylgruppe mit 12 oder 14 C-Atomen und x = 2 ist und als Monoisopropanolammoniumsalz vorliegt.
Weitere besonders bevorzugte erfindungsgemäße Zusammensetzungen sind dadurch gekennzeichnet, dass das mindestens eine zusätzliche anionische Tensid ausgewählt ist aus Alkylsulfaten mit 8 - 18 C-Atomen in der Alkylgruppe, Alkylpolyglycolethersulfaten und Ethercarbonsäuren, jeweils mit 8 - 18 C-Atomen in der Alkylgruppe und 1 - 12 Glycolethergruppen im Molekül, Sulfobernsteinsäuremono- und -dialkylestern mit 8 - 18 C-Atomen in der Alkylgruppe, Sulfobernsteinsäuremonoalkylpolyoxyethylestern mit 8 - 18 C-Atomen in der Alkylgruppe und 1 - 6 Glycolethergruppen im Molekül, Monoglyceridsulfaten, Alkyl- und Alkenylphosphaten, Alkyl- und Alkenyletherphosphaten sowie Eiweißfettsäurekondensaten, jeweils mit 8 - 18 C-Atomen in der Alk(en)ylgruppe beziehungsweise in der Acylgruppe und gegebenenfalls 1 - 10 Glycolethergruppen im Molekül.
Weitere besonders bevorzugte erfindungsgemäße Zusammensetzungen sind dadurch gekennzeichnet, dass das mindestens eine weitere anionische Tensid in einer Gesamtmenge von 0,1 - 5 Gew.-%, bevorzugt 0,5 - 2 Gew.-%, besonders bevorzugt 1 - 1,5 Gew.-%, jeweils bezogen auf das Gewicht der gesamten Zusammensetzung, enthalten ist.

Die erfindungsgemäßen Seifenzubereitungen weisen bereits ein gutes Schaumvermögen auf. Um den hohen Ansprüchen der Verbraucher an ein besonders schnelles Anschäumen gerecht zu werden, bestand weiterhin die Aufgabe, eine konzentrierte Seifenzubereitung bereitzustellen, die sich nicht nur einfach herstellen lässt und eine hohe Lagerstabilität und einen attraktiven Perlglanz aufweist, sondern die auch ein besonders schnelles Anschäumverhalten und/oder eine hohe Schaumstabilität zeigt und gleichzeitig eine hohe Hautverträglichkeit aufweist. Es wurde gefunden, dass durch die Zugabe eines zusätzlichen nichtionischen Tensids, das in wässriger Lösung ein gutes Anschäumverhalten aufweist und einen stabilen Schaum bildet, Schaumvermögen und Hautverträglichkeit der erfindungsgemäßen Zusammensetzungen weiter optimiert werden konnten. Unter Optimierung des Anschäumverhaltens wird erfindungsgemäß verstanden, dass innerhalb der ersten Sekunden, in denen die Seifenzubereitung angerührt wird, eine starke Schaumbildung einsetzt und dieser "Flash foam" sein Volumen auch für einige Zeit hält und nicht sofort wieder zusammenfällt. Insbesondere war überraschend, dass hierzu schon relativ geringe Mengen des nichtionischen Tensids ausreichen. Weitere bevorzugte erfindungsgemäße Zusammensetzungen sind daher dadurch gekennzeichnet, dass mindestens ein nichtionisches Tensid enthalten ist.

Erfindungsgemäß bevorzugte nichtionische Tenside sind
- Aminoxide,
- Fettsäuredialkanolamide,
- Alk(en)yloligoglycoside entsprechend der allgemeinen Formel RO-(Z)ₓ wobei R für Alkyl oder Alkenyl, Z für Zucker sowie x für die Anzahl der Zuckereinheiten steht. Die erfindungsgemäß verwendbaren Alk(en)yloligoglycoside können lediglich einen bestimmten Alk(en)ylrest R enthalten. Üblicherweise werden diese Verbindungen aber ausgehend von natürlichen Fetten und Ölen oder Mineralölen hergestellt. In diesem Fall liegen als Alk(en)ylreste R Mischungen entsprechend den Ausgangsverbindungen bzw. entsprechend der jeweiligen Aufarbeitung dieser Verbindungen vor. Besonders bevorzugt sind solche Alk(en)yloligoglycoside, bei denen R
- im wesentlichen aus C₈- und C₁₀-Alk(en)ylgruppen oder
- im wesentlichen aus C₁₂- und C₁₄-Alk(en)ylgruppen oder
- im wesentlichen aus C₈- bis C₁₆-Alk(en)ylgruppen oder
- im wesentlichen aus C₁₂- bis C₁₆-Alk(en)ylgruppen oder
- im wesentlichen aus C₁₆- bis C₁₈-Alk(en)ylgruppen besteht.
   Bevorzugt sind Alk(en)ylreste mit 8 - 18 C-Atomen. Besonders bevorzugt sind gesättigte Alkylreste mit 8 - 18 C-Atomen. Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt, beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.
   Die erfindungsgemäß verwendbaren Alk(en)yloligoglycoside enthalten durchschnittlich 1,1 bis 5 Zuckereinheiten. Alk(en)yloligoglycoside mit x-Werten (= Oligomerisierungsgrad) von 1,1 bis 2,0 sind bevorzugt. Besonders bevorzugt sind Alk(en)yloligoglycoside, bei denen x 1,1 bis 1,8 beträgt. Entsprechende Handelsprodukte sind beispielsweise unter den Bezeichnungen Plantaren oder Plantacare von der Firma Cognis erhältlich.
   Auch die alkoxylierten Homologen der genannten Alk(en)yloligoglycoside können erfindungsgemäß eingesetzt werden. Diese Homologen können durchschnittlich bis zu 10 Ethylenoxid- und/oder Propylenoxideinheiten pro Alk(en)ylglycosideinheit enthalten;
- Fettsäure-N-alkylglucamide.

Besonders bevorzugte nichtionische Tenside sind Diethanolamide von linearen C₆ - C₁₈-Alkancarbonsäuren und deren Mischungen, bevorzugt von linearen C₈ - C₁₈-Alkancarbonsäuren und deren Mischungen, insbesondere Lauramide DEA und Cocamide DEA.
Weitere besonders bevorzugte erfindungsgemäße Zusammensetzungen sind dadurch gekennzeichnet, dass das mindestens eine nichtionische Tensid ausgewählt ist aus Alk(en)yloligoglycosiden mit 8 - 18 C-Atomen in der Alk(en)ylgruppe, Fettsäurediethanolamiden und Aminoxiden sowie Mischungen hiervon.

Weitere besonders bevorzugte erfindungsgemäße Zusammensetzungen sind dadurch gekennzeichnet, dass das mindestens eine weitere nichtionische Tensid in einer Gesamtmenge von 0,1 - 2 Gew.-%, bevorzugt 0,5 - 1,5 Gew.-%, besonders bevorzugt 0,7 - 1,0 Gew.-%, jeweils bezogen auf das Gewicht der gesamten Zusammensetzung, enthalten ist.

Wie bereits erwähnt, ist die Viskosität eine wichtige Kenngröße der erfindungsgemäßen Zusammensetzungen. Durch den Zusatz der erfindungsgemäß ausgewählten Elektrolyten in den erfindungsgemäß ausgewählten Mengen zum Fettsäure-Neutralisierungsmittel-Wasser-Gemisch bleibt die Reaktionsmischung auch bei den Temperaturen, die zum Aufschmelzen der Fettsäuren notwendig ist, so viskos, dass sie mit üblichen Rührapparaturen in zufriedenstellender Weise homogenisiert werden kann. Die Endviskosität sollte sich nach dem Herstellungs- und Abkühlprozess relativ schnell, das heißt, innerhalb von 2 bis 5 Tagen, einstellen, da die notwendige Zwischenlagerung den Herstellprozess verteuert. Die Viskosität sollte so sein, dass das Produkt einfach in Tuben oder Pumpspender abgefüllt und aus diesen später, das heißt, beim Endverbraucher, wieder entnommen werden kann. Die Viskosität sollte auch bei einer (unsachgemäßen und nicht erwünschten) Lagerung bei Temperaturen von 35 °C und darüber nicht zu stark ansteigen und bei einem anschließenden Abkühlen und Temperieren bei 20 - 25°C möglichst wieder den ursprünglichen Wert annehmen, um weiterhin aus der Verpackung dosierbar zu sein. Bevorzugte erfindungsgemäße Zusammensetzungen sind gekennzeichnet durch eine Viskosität von 80.000 - 300.000 mPas, bevorzugt 120.000 - 200.000 mPas, jeweils gemessen 5 Tage nach Herstellung, bei 20°C Umgebungs- und Probentemperatur mit einem Brookfield Rotationsviskosimeter Modell RVT, mit Helipath, Spindel TD, 5 Umdrehungen pro Minute.

Die erfindungsgemäßen Zusammensetzungen stellen in jeglicher Hinsicht überaus befriedigende Präparate dar. Die erfindungsgemäßen Zusammensetzungen und daraus erhältliche Schäume weisen sehr gute sensorische Eigenschaften auf, wie beispielsweise Verteilbarkeit auf der Haut, sensorischer Eindruck, Volumen und Konsistenz des erzeugten Schaums und - besonders wichtig für die Verwendung als Rasierhilfsmittel - gutes Gleitvermögen der Rasierklinge über die Haut ("lubricity"). Aus den erfindungsgemäßen Zusammensetzungen sind feinblasige, reichhaltige, cremige Schäume von hervorragender kosmetischer Eleganz erhältlich. Weiterhin sind aus den erfindungsgemäßen Zusammensetzungen besonders gut hautverträgliche Zusammensetzungen erhältlich, die sich besonders gut auf der Haut verteilen lassen.

In einer weiteren bevorzugten Ausführungsform können die erfindungsgemäßen Zusammensetzungen durch den Zusatz von mindestens einem hochmolekularen Polyethylenglycol, bestehend aus Ethylenoxid-Einheiten, weiter optimiert werden. Ein Zusatz an hochmolekularen Polyethylenglycolen verdickt die Zusammensetzung und bildet auf der Haut einen Film, der bei der Rasur das Gleiten der Klinge über die Haut verbessert und somit die Rasur erleichtert. Außerdem verleihen hochmolekulare Polyethylenglycole der Zusammensetzung ein angenehmes Hautgefühl. Ein zu hoher Gehalt an hochmolekularen Polyethylenglycolen führt allerdings zu einer zu hohen Elastizität der Zusammensetzung, was z. B. zu Fädenziehen und einer schlechteren Verteilbarkeit auf der Haut führen kann. Auch kann ein zu hoher Gehalt an hochmolekularen Polyethylenglycolen die Qualität und das Hautgefühl des Schaumes beeinträchtigen. Ein zu hoher Gehalt an hochmolekularen Polyethylenglycolen führt außerdem zu Problemen bei der Herstellung, da sich derartige elastische Zusammensetzungen nur unter größerem Energieeintrag homogenisieren und anschließend nur langsam abfüllen lassen.
Weitere besonders bevorzugte erfindungsgemäße Zusammensetzungen enthalten mindestens ein Polyethylenglycol mit einem mittleren Molekulargewicht von mindestens 300.000 Dalton in einer Menge von 0,001 - 0,2 Gew.-%, bezogen auf die Zusammensetzung. Dieses Polyethylenglycol mit einem mittleren Molekulargewicht von mindestens 300.000 Dalton weist die allgemeine Strukturformel H-(OCH₂CH₂)ₙ-OH auf.
Derartige Polyethylenglycole sind zum Beispiel ein Polyethylenglycol mit einem mittleren Molekulargewicht von 600.000 Dalton mit der INCI-Bezeichnung PEG-14M, erhältlich z. B. unter den Handelsnamenen Polyox WSR N-3000 oder Polyox WSR 205 von Amerchol, ein Polyethylenglycol mit einem mittleren Molekulargewicht von 1.000.000 Dalton mit der INCI-Bezeichnung PEG-23M, erhältlich z. B. unter dem Handelsnamen Polyox WSR N-12K von Amerchol, ein Polyethylenglycol mit einem mittleren Molekulargewicht von 2.000.000 Dalton mit der INCI-Bezeichnung PEG-45M, erhältlich z. B. unter dem Handelsnamen Polyox WSR N-60K von Amerchol, ein Polyethylenglycol mit einem mittleren Molekulargewicht von 4.000.000 Dalton mit der INCI-Bezeichnung PEG-90M, erhältlich z. B. unter dem Handelsnamen Polyox WSR 301 von Amerchol, ein Polyethylenglycol mit einem mittleren Molekulargewicht von 5.000.000 Dalton mit der INCI-Bezeichnung PEG-115M, erhältlich z. B. unter dem Handelsnamen Polyox Coagulant von Amerchol.
Bevorzugt sind Polyethylenglycole mit einem mittleren Molekulargewicht von 600.000 - 7.000.000 Dalton, besonders bevorzugt sind Polyethylenglycole mit einem mittleren Molekulargewicht von 2.000.000 - 5.000.000 Dalton, außerordentlich bevorzugt ist ein Polyethylenglycol mit einem mittleren Molekulargewicht von 4.000.000 Dalton. Es kann erfindungsgemäß bevorzugt sein, Mischungen der genannten hochmolekularen Polyethylenglycole einzusetzen.
Das mindestens eine Polyethylenglycol mit einem mittleren Molekulargewicht von mindestens 300.000 Dalton ist bevorzugt in einer Gesamtmenge von 0,001 - 0,2 Gew.-%, besonders bevorzugt 0,005 - 0,1 Gew.-% und außerordentlich bevorzugt 0,008 - 0,05 Gew.-%, jeweils bezogen auf die gesamte Zusammensetzung, enthalten. Besonders bevorzugt sind Konzentrationen von 0,009, 0,01 und 0,015 Gew.-%, jeweils bezogen auf die gesamte Zusammensetzung. Es war überraschend und für den Fachmann nicht vorauszusehen, dass mit derartig geringen Konzentrationen ein gutes Gleiten der Klinge auf der Haut und insgesamt ein hervorragendes Hautgefühl erzielt und gleichzeitig die Klebrigkeit der Zusammensetzung herabgesetzt und die Qualität des erzeugten Schaums verbessert werden konnte.

Weitere erfindungsgemäß bevorzugte Zusammensetzungen sind dadurch gekennzeichnet, dass sie wenigstens ein Polyalkylenglycol-modifiziertes Silicon enthalten. Polyalkylenglycol-modifizierte Silicone reduzieren die Klebrigkeit und hinterlassen ein frisches Hautgefühl. Darüber hinaus wurde überraschend festgestellt, dass durch den Zusatz von mindestens einem Feuchthaltemittel, wie vorstehend aufgeführt, Verbesserungen bei der Herstellung, wie verkürzte Zeit bis zur Einstellung des Perlglanzes und/oder bis zur Stabilisierung der Viskosität, erzielt werden konnten. Unter Polyalkylenglycol-modifizierten Siliconen werden erfindungsgemäß Polyorganosiloxane mit Polyethylenglycol-, Polypropylenglycol- oder Polyethylenglycol/Polypropylenglycol-End- oder Seitenketten verstanden. Erfindungsgemäß besonders bevorzugt sind sogenannte Silicon-Copolyole, insbesondere solche Verbindungen mit der früheren INCI-Bezeichnung Dimethicone Copolyol, die beispielsweise von Wacker-Chemie unter der Bezeichnung Belsil^{®} DMC 6031, Belsil^{®} DMC 6032, Belsil^{®} DMC 6038 oder Belsil^{®} DMC 3071 VP bzw. von Dow Corning unter der Bezeichnung DC 2501 bzw. von Degussa unter der Bezeichnung Abil EM 97 im Handel sind. Erfindungsgemäß kann auch ein beliebiges Gemisch dieser Silicone eingesetzt werden. Weitere hydrophil modifizierte Silicon-Copolyole sind ausgewählt aus den Poly-(C₂-C₃)-alkylenglycol-modifizierten Siliconen, deren frühere INCI-Bezeichnung Dimethicone Copolyol lautete, mit den aktuellen INCI-Bezeichnungen PEG-x Dimethicone (mit x = 2 - 20, bevorzugt 3 - 17, besonders bevorzugt 11 - 12), Bis-PEG-y Dimethicone (mit y = 3 - 25, bevorzugt 4 - 20), PEG/PPG a/b Dimethicone (wobei a und b unabhängig voneinander für Zahlen von 2 - 30, bevorzugt 3 - 30 und besonders bevorzugt 12 - 20, insbesondere 14 - 18, stehen), Bis-PEG/ PPG-c/d Dimethicone (wobei c und d unabhängig voneinander für Zahlen von 10 - 25, bevorzugt 14 - 20 und besonders bevorzugt 14 - 16, stehen) und Bis-PEG/PPG-e/f PEG/ PPG g/h Dimethicone (wobei e, f, g und h unabhängig voneinander für Zahlen von 10 - 20, bevorzugt 14 - 18 und besonders bevorzugt 16, stehen). Beispiele für derartige Silicone sind PEG/PPG-18/18 Dimethicone, das in einer 1:9-Mischung mit Cyclomethicone unter der Bezeichnung DC 3225 C bzw. DC 5225 C von Dow Corning im Handel erhältlich ist, sowie Bis-PEG/PPG-14/14 Dimethicone, das in einer Mischung mit Cyclomethicone unter der Bezeichnung Abil EM 97 (Goldschmidt) im Handel erhältlich ist. Erfindungsgemäß besonders geeignet sind PEG-x Dimethicone mit x = 2 - 20, bevorzugt x = 3 -17, besonders bevorzugt x = 11 - 12, außerordentlich bevorzugt ist das Handelsprodukt Dow Corning 193 Surfactant mit der INCI-Bezeichnung PEG-12 Dimethicone.
Weiterhin bevorzugt sind Poly-(C₂-C₃)alkylenglycol-modifizierte Silicone, die mit C₄-C₁₈-Alkylgruppen hydrophob modifiziert sind, besonders bevorzugt Cetyl PEG/PPG-10/1 Dimethicone (früher: Cetyl Dimethicone Copolyol, erhältlich unter der Bezeichnung Abil EM 90), weiterhin Alkyl Methicone Copolyole und Alkyl Dimethicone Ethoxy Glucoside.
Die Gesamtmenge des/der Polyalkylenglycol-modifizierten Silicons/Silicone in den erfindungsgemäßen Zusammensetzungen beträgt bevorzugt 0,1 - 8 Gew.-%, besonders bevorzugt 0,5 - 5 Gew.-% und außerordentlich bevorzugt 1 - 3 Gew.-%, jeweils bezogen auf das Gesamtgewicht der Zusammensetzung.

In einer weiteren bevorzugten Ausführungsform können die erfindungsgemäßen Zusammensetzungen zur Verbesserung ihrer hautpflegenden Wirkung mindestens ein Öl und/oder mindestens einen Fettstoff enthalten, das/der keine Estergruppe im Molekül aufweist. Erfindungsgemäß besonders bevorzugte Öle und Fettstoffe ohne Estergruppe im Molekül sind ausgewählt aus
- flüssigen Paraffinölen, Isoparaffinölen und synthetischen Kohlenwasserstoffen, z. B. 1,3-Di-(2-ethyl-hexyl)-cyclohexan (Cetiol^{®} S), Polydecen oder Polyisobuten,
- Di-n-alkylethern mit insgesamt 12 bis 36, insbesondere 12 bis 24 C-Atomen, z. B. Di-n-octylether (Cetiol^{®} OE), n-Hexyl-n-octylether und n-Octyl-n-decylether,
- Siliconverbindungen, bevorzugt ausgewählt aus Decamethylcyclopentasiloxan, Dodecamethylcyclohexasiloxan und linearen Siliconpolymeren, die gewünschtenfalls quervernetzt sein können, z. B. Polydialkylsiloxanen wie bevorzugt Hexamethyldisiloxan (L₂), Octamethyltrisiloxan (L₃), 3-Hexylheptamethyltrisiloxan (z. B. Dow Corning 2-1731), Decamethyltetrasiloxan (L₄), Dodecamethylpentasiloxan (L₅), Polyalkylarylsiloxanen, zum Beispiel Poly(methylphenylsiloxan), sowie Mischungen der vorgenannten Substanzen, insbesondere bevorzugt Mischungen aus L₃ und L₄, wie sie unter der Bezeichnung Dow Corning 2-1184 erhältlich sind, weiterhin bevorzugt die unter der Bezeichnung Dow Corning 200 Fluid erhältlichen Polymere mit unterschiedlicher Kettenlänge und Viskosität (INCI-Bezeichnung : Dimethicone).
Besonders bevorzugte Öle sind flüssige Paraffinöle, Isoparaffinöle und synthetische Kohlenwasserstoffe, außerordentlich bevorzugt sind Paraffinöle.
Bevorzugte erfindungsgemäße Zusammensetzungen sind dadurch gekennzeichnet, dass sie mindestens ein Öl und/oder mindestens einen Fettstoff ohne Estergruppe im Molekül, bevorzugt mindestens ein Paraffinöl, oder Mischungen hiervon insgesamt in Mengen von 0,1 - 4 Gew.-%, besonders bevorzugt 0,5 - 3,5 Gew.-% und außerordentlich bevorzugt 1- 3 Gew.-%, jeweils bezogen auf das Gesamtgewicht der Zusammensetzung, enthalten.

Weiterhin wurde überraschend festgestellt, dass durch einen Gehalt an mindestens einer Kieselsäure die rheologischen und/oder sensorischen Eigenschaften der erfindungsgemäßen Zusammensetzungen in für den Fachmann nicht zu erwartendem Ausmaß verbessert werden konnten. In einer weiteren bevorzugten Ausführungsform enthalten die erfindungsgemäßen Zusammensetzungen daher zur Verbesserung der rheologischen und/oder sensorischen Eigenschaften mindestens eine Kieselsäure. Die Kieselsäure ist ausgewählt aus pyrogenen und Fällungskieselsäuren sowie Mischungen hiervon, wobei Fällungskieselsäuren erfindungsgemäß besonders bevorzugt sind. Besonders bevorzugte erfindungsgemäße Zusammensetzungen sind dadurch gekennzeichnet, dass sie Kieselsäure insgesamt in Mengen von 0,01 - 1 Gew.-%, bevorzugt 0,1 - 0,7 Gew.-% und besonders bevorzugt 0,2 - 0,5 Gew.-%, jeweils bezogen auf das Gesamtgewicht der Zusammensetzung, enthalten.

In einer weiteren bevorzugten Ausführungsform können die erfindungsgemäßen Zusammensetzungen zur Verbesserung ihrer hauterfrischenden Wirkung mindestens einen hautkühlenden Wirkstoff enthalten. Erfindungsgemäß geeignete hautkühlende Wirkstoffe sind beispielsweise ausgewählt aus Menthol, Isopulegol sowie Mentholderivaten, z. B. Menthyllactat, Menthylpyrrolidoncarbonsäure, Menthylmethylether, Menthoxypropandiol, Mentholethylenglycolcarbonat, Mentholpropylenglycolcarbonat, Menthonglycerinacetal (9-Methyl-6-(1-methylethyl)-1,4-dioxaspiro(4.5)decan-2-methanol), Monomenthylsuccinat und 2-Hydroxymethyl-3,5,5-trimethylcyclohexanol. Besonders bevorzugt sind Menthol, Menthonglycerinacetal (9-Methyl-6-(1-methylethyl)-1,4-dioxaspiro(4.5)-decan-2-methanol), Isopulegol, Mentholethylenglycolcarbonat, Mentholpropylenglycolcarbonat und Menthoxypropandiol sowie Mischungen dieser Substanzen.
Besonders bevorzugte erfindungsgemäße Zusammensetzungen sind dadurch gekennzeichnet, dass sie mindestens einen hautkühlenden Wirkstoff in Mengen von insgesamt 0,01 - 1 Gew.-%, bevorzugt 0,02 - 0,5 Gew.-% und besonders bevorzugt 0,1 - 0,3 Gew.-%, jeweils bezogen auf das Gesamtgewicht der Zusammensetzung, enthalten.

Besonders bevorzugte erfindungsgemäße Zusammensetzungen sind dadurch gekennzeichnet, dass sie mindestens einen hautfeuchthaltenden Wirkstoff enthalten. Besonders bevorzugte hautfeuchthaltende Wirkstoffe sind ausgewählt aus Aloe vera, Aminosäuren, deren Salzen und Estern, insbesondere Chitosoniumpyrrolidoncarbonsäuresalze, Proteinen, Proteinhydrolysaten, Pyrrolidoncarbonsäure, weiteren Bestandteilen des "Natural Moisturizing Factor", der aus 40% freien Aminosäuren, 12% Pyrrolidoncarbonsäure, 12% Lactaten, 7% Harnstoff, 1,5% Harnsäure sowie D-Glucosamin, Kreatinin und verschiedenen Salzen besteht, Desoxyzuckern, besonders bevorzugt Rhamnose und Fucose, Polysacchariden, die mindestens einen Desoxyzucker-Baustein enthalten, besonders bevorzugt aus den Handelsprodukten Fucogel^{®} (INCI-Bezeichnung Biosaccharide Gum-1) von Solabia, Rhamnosoft^{®} (INCI-Bezeichnung Biosaccharide Gum-2) von Solabia, Fucogenol^{®} (INCI-Bezeichnung Biosaccharide Gum-3) von Solabia und Glycofilm^{®} (INCI-Bezeichnung Biosaccharide Gum-4) von Solabia, weiterhin Mischungen der vorgenannten, mindestens einen Desoxyzucker-Baustein enthaltenden Polysaccharide, beispielsweise der Mischung aus Biosaccharide Gum-2 und Biosaccharide Gum-3, erhältlich als Handelsprodukt Elastinol plus^{®} von Solabia, weiterhin Harnstoff, N,N'-Bis(2-hydroxyethyl)harnstoff, Betain (Me₃N⁺-CH₂-COO⁻), Glycosaminoglycanen, besonders bevorzugt Hyaluronsäure, Dextran, Dextransulfat, Chondroitin-4-sulfat und Chondroitin-6-sulfat, sowie beliebigen Mischungen dieser Substanzen.
Erfindungsgemäß besonders bevorzugte Zusammensetzungen sind dadurch gekennzeichnet, dass sie mindestens einen hautfeuchthaltenden Wirkstoff in einer Gesamtmenge von 0,001 bis 10 Gew.-%, besonders bevorzugt 0,01 bis 5 Gew.-% und außerordentlich bevorzugt 0,1 bis 1 oder 2 Gew.-%, jeweils bezogen auf die gesamte Zusammensetzung, enthalten.

Erfindungsgemäß besonders bevorzugte Zusammensetzungen sind dadurch gekennzeichnet, dass sie weiterhin mindestens einen hautberuhigenden Wirkstoff enthalten. Erfindungsgemäß bevorzugte hautberuhigende Wirkstoffe sind ausgewählt aus Panthenol, Allantoin, α-Bisabolol, α-Liponsäure, Extrakten aus Centella asiatica, beispielsweise erhältlich unter der Bezeichnung Madecassicoside von DSM, Glycyrrethinsäure, die besonders bevorzugt in Liposomen verkapselt vorliegt und in dieser Form z. B. unter dem Handelsnamen Calmsphere von Soliance erhältlich ist, Mischungen aus Getreidewachsen, Extrakten aus Schibutter und Argania Spinosa-Öl mit der INCI-Bezeichnung "Spent grain wax and Butyrospermum Parkii (shea butter) extract and Argania Spinosa Kernel Oil", wie sie z. B. unter der Handelsbezeichnung Stimu-Tex AS von der Firma Pentapharm erhältlich sind, Extrakten aus Vanilla Tahitensis, wie sie z. B. unter der Handelsbezeichnung Vanirea (INCI : Vanilla Tahitensis Fruit Extract) von der Firma Solabia erhältlich sind, Alginhydrolysaten, wie sie z. B. unter der Handelsbezeichnung Phycosaccharide, insbesondere Phycosaccharide Al, von der Firma Codif erhältlich sind, Extrakten aus Bacopa Monniera, wie sie z. B. unter der Handelsbezeichnung Bacocalmine von der Firma Sederma erhältlich sind, Extrakten aus der Rooibos-Pflanze, wie sie z. B. unter dem Handelsnamen Rooibos Herbasec MPE von der Firma Cosmetochem erhältlich sind, Hefeextrakten, besonders bevorzugt das Handelsprodukt Drieline (INCI-Bezeichnung "Sorbitol, Yeast Extract"), erhältlich von der Firma Lanatech, den physiologisch verträglichen Salzen von Sterolsulfaten, wie sie z. B. unter der Handelsbezeichnung Phytocohesine (INCI: Sodium Beta-Sitosterylsulfate) von der Firma Vincience erhältlich sind, Aminodicarbonsäuren mit einer C-Kettenlänge von 3 - 6 Kohlenstoffatomen sowie deren physiologisch verträglichen Salzen, bevorzugt ausgewählt aus Aminomalonsäure, Aminobernsteinsäure (= Asparaginsäure), Aminoglutarsäure und Aminoadipinsäure sowie deren physiologisch verträglichen Salzen wie Kaliumaspartat und Magnesiumaspartat, sowie beliebigen Mischungen dieser Substanzen.

Erfindungsgemäß besonders bevorzugte Zusammensetzungen sind dadurch gekennzeichnet, dass sie mindestens einen hautberuhigenden Wirkstoff in einer Gesamtmenge von 0,001 - 5 Gew.-%, besonders bevorzugt 0,01 - 2 Gew.-% und außerordentlich bevorzugt 0,1 - 1 Gew.-%, jeweils bezogen auf die gesamte Zusammensetzung, enthalten.

Erfindungsgemäße Zusammensetzungen können bereits durch leichtes Verreiben, beispielsweise in den Händen oder beim Auftragen und Verreiben auf der Haut, aber auch durch Rühren oder sonstige Aufschäumvorgänge zu Schäumen gestaltet werden.

Ein weiterer Gegenstand der vorliegenden Erfindung ist die nicht-therapeutische Verwendung einer kosmetischen Zusammensetzung, die Wasser, mindestens eine Monocarbonsäure mit 8 - 24 Kohlenstoffatomen, ausgewählt aus linearen, verzweigten, gesättigten und ungesättigten Monocarbonsäuren mit 8 - 24 Kohlenstoffatomen, mindestens ein Neutralisierungsmittel für die Monocarbonsäure/n, sowie mindestens einen Elektrolyten, der ausgewählt ist aus Natriumchlorid, Natriumsulfat, Natriumlactat, Natriumglycolat, Natriumcitrat, Kaliumchlorid, Kaliumsulfat, Magnesiumchlorid, Calciumchlorid sowie Mischungen hiervon, in einer Gesamtmenge von 0,5 - 10 Gew.-%, bezogen auf das Gewicht der gesamten Zusammensetzung, enthält, als Rasierhilfsmittel oder als Reinigungsmittel für die Haut und/oder das Haar.
Ein weiterer Gegenstand der vorliegenden Erfindung ist die Verwendung einer Zusammensetzung, die Wasser, mindestens eine Monocarbonsäure mit 8 - 24 Kohlenstoffatomen, ausgewählt aus linearen, verzweigten, gesättigten und ungesättigten Monocarbonsäuren mit 8 - 24 Kohlenstoffatomen, mindestens ein Neutralisierungsmittel für die Monocarbonsäure/n, sowie mindestens einen Elektrolyten, der ausgewählt ist aus Natriumchlorid, Natriumsulfat, Natriumlactat, Natriumglycolat, Natriumcitrat, Kaliumchlorid, Kaliumsulfat, Magnesiumchlorid, Calciumchlorid sowie Mischungen hiervon, in einer Gesamtmenge von 0,5 - 10 Gew.-%, bezogen auf das Gewicht der gesamten Zusammensetzung, enthält, zur Herstellung einer topischen Zusammensetzung zur therapeutischen Behandlung der Haut, der Schleimhäute und/oder der Kopfhaut.
Ein weiterer Gegenstand der vorliegenden Erfindung ist die nicht-therapeutische Verwendung einer kosmetischen Zusammensetzung, die Wasser, mindestens eine Monocarbonsäure mit 8 - 24 Kohlenstoffatomen, ausgewählt aus linearen, verzweigten, gesättigten und ungesättigten Monocarbonsäuren mit 8 - 24 Kohlenstoffatomen, mindestens ein Neutralisierungsmittel für die Monocarbonsäure/n, sowie mindestens einen Elektrolyten, der ausgewählt ist aus Natriumchlorid, Natriumsulfat, Natriumlactat, Natriumglycolat, Natriumcitrat, Kaliumchlorid, Kaliumsulfat, Magnesiumchlorid, Calciumchlorid sowie Mischungen hiervon, in einer Gesamtmenge von 0,5 - 10 Gew.-%, bezogen auf das Gewicht der gesamten Zusammensetzung, enthält, zur Befeuchtung und/oder als Moisturizer für die Haut und/oder das Haar.

Ein weiterer Gegenstand der vorliegenden Erfindung ist die nicht-therapeutische Verwendung einer kosmetischen Zusammensetzung, die Wasser, mindestens eine Monocarbonsäure mit 8 - 24 Kohlenstoffatomen, ausgewählt aus linearen, verzweigten, gesättigten und ungesättigten Monocarbonsäuren mit 8 - 24 Kohlenstoffatomen, mindestens ein Neutralisierungsmittel für die Monocarbonsäure/n, sowie mindestens einen Elektrolyten, der ausgewählt ist aus Natriumchlorid, Natriumsulfat, Natriumlactat, Natriumglycolat, Natriumcitrat, Kaliumchlorid, Kaliumsulfat, Magnesiumchlorid, Calciumchlorid sowie Mischungen hiervon, in einer Gesamtmenge von 0,5 - 10 Gew.-%, bezogen auf das Gewicht der gesamten Zusammensetzung, enthält, zur Erfrischung für die Haut und/oder das Haar.

Ein weiterer Gegenstand der vorliegenden Erfindung ist die nicht-therapeutische Verwendung einer kosmetischen Zusammensetzung, die Wasser, mindestens eine Monocarbonsäure mit 8 - 24 Kohlenstoffatomen, ausgewählt aus linearen, verzweigten, gesättigten und ungesättigten Monocarbonsäuren mit 8 - 24 Kohlenstoffatomen, mindestens ein Neutralisierungsmittel für die Monocarbonsäure/n, sowie mindestens einen Elektrolyten, der ausgewählt ist aus Natriumchlorid, Natriumsulfat, Natriumlactat, Natriumglycolat, Natriumcitrat, Kaliumchlorid, Kaliumsulfat, Magnesiumchlorid, Calciumchlorid sowie Mischungen hiervon, in einer Gesamtmenge von 0,5 - 10 Gew.-%, bezogen auf das Gewicht der gesamten Zusammensetzung, enthält, als anti-irritatives Rasierhilfsmittel und/oder anti-irritatives Reinigungsmittel für die Haut und/oder das Haar.

Ein weiterer Gegenstand der vorliegenden Erfindung ist ein nicht-therapeutisches, kosmetisches Verfahren zur Rasur und/oder zur Reinigung der Haut und/oder des Haars, bei dem eine kosmetische Zusammensetzung, die Wasser, mindestens eine Monocarbonsäure mit 8 - 24 Kohlenstoffatomen, ausgewählt aus linearen, verzweigten, gesättigten und ungesättigten Monocarbonsäuren mit 8 - 24 Kohlenstoffatomen, mindestens ein Neutralisierungsmittel für die Monocarbonsäure/n, sowie mindestens einen Elektrolyten, der ausgewählt ist aus Natriumchlorid, Natriumsulfat, Natriumlactat, Natriumglycolat, Natriumcitrat, Kaliumchlorid, Kaliumsulfat, Magnesiumchlorid, Calciumchlorid sowie Mischungen hiervon, in einer Gesamtmenge von 0,5 - 10 Gew.-%, bezogen auf das Gewicht der gesamten Zusammensetzung, enthält, auf die Haut und/oder die Haare aufgetragen und anschließend gewünschtenfalls abgespült und/oder abgewischt wird. Das Entfernen der Zusammensetzung kann nach einer Einwirkzeit von ca. 5 Sekunden bis 20 Minuten oder länger erfolgen. Für die Rasur bevorzugt ist eine Einwirkzeit von 10 bis 90 Sekunden.

Die erfindungsgemäßen Zusammensetzungen können ferner gegebenenfalls in der Kosmetik übliche Zusatzstoffe, beispielsweise Parfüms, Deodorantwirkstoffe, antimikrobielle Stoffe, Komplexierungs- und Sequestrierungsmittel, Antioxidantien, Pflanzenextrakte, insbesondere adstringierende Pflanzenextrakte, andere adstringierende und/oder porenverfeinernde und/oder porenverkleinernde Wirkstoffe, z. B. Aluminiumsalze, weiterhin Vitamine und Vitaminderivate, bevorzugt Retinylpalmitat, Niacinamid oder Tocopherylacetat, Mineralwässer, mineralische Salze, Konservierungsmittel, Bakterizide, Schaumstabilisatoren, organische Lösungsmittel, insbesondere Ethanol, Farbstoffe und/oder Pigmente enthalten. Die Einsatzkonzentration dieser Zusatzstoffe, insbesondere der Elektrolyten, muss selbstverständlich so gewählt sein, dass sie die erfindungsgemäße Lehre nicht beeinträchtigt

Die nachfolgenden Beispiele sollen die vorliegende Erfindung verdeutlichen, ohne sie hierauf einzuschränken. Alle Mengenangaben sind in Gew.-%, soweit nicht anders angegeben.

Die Herstellung der erfindungsgemäßen Zusammensetzungen wird bevorzugt in der Weise durchgeführt, dass man Natrium- oder Kaliumhydroxid oder ein Gemisch von Natrium- und Kaliumhydroxid, das zusammen mit dem/den Elektrolyten d) in Wasser gelöst wurde, unter Rühren in einen Behälter einträgt, in dem für den ersten Verseifungsschritt etwa 65 Gew.-% des gesamten Gehalts an Monocarbonsäuren mit 8 - 24 Kohlenstoffatomen b), die als solche zugegeben werden, und die gesamte Menge des gegebenenfalls vorhandenen Glyceryltriesters von Monocarbonsäuren mit 8 - 24 Kohlenstoffatomen flüssig bei 50 bis 90°C, insbesondere 75 - 90°C, vorgelegt worden sind. Gewünschtenfalls vorhandene Polyole, wie Glycerin, werden zusammen mit der Natron- oder Kalilauge zudosiert, bzw. sie entstehen in situ aus dem Glyceryltriester, sofern vorhanden. Dieser erste Verseifungsschritt läuft bei 75 - 90°C ab.
Für den zweiten Verseifungsschritt wird die restliche Menge an Monocarbonsäuren mit 8 - 24 Kohlenstoffatomen b), die als solche zugegeben werden, zudosiert. Dieser zweite Verseifungsschritt läuft bei 90°C ab.
Während dieses zweiten Verseifungsschritts steigt die Viskosität der Reaktionsmischung sehr stark an. Hier macht sich der Einfluss der erfindungsgemäß verwendeten Elektrolyte bemerkbar.
Anschließend wird die Reaktionsmischung auf 45 °C abgekühlt, gegebenenfalls werden bei dieser Temperatur weitere Tenside (Texapon CS etc.) zugegeben.

Im Anschluss wird die Reaktionsmischung auf 30 °C abgekühlt, und Duftstoffe sowie gegebenenfalls weitere Hilfsmittel werden nacheinander untergemischt.

Die Entwicklung des Perlglanzes beginnt am Schluss dieses Herstellungsprozesses und setzt sich während der folgenden Stunden und Tage fort.

**Tabelle 1: Erfindungsgemäße Rasiercremes (Angaben in Gew.-%)**

| | Nr. 1 a-d | Nr. 2 a-b | Nr. 3 | Vergleichsbeispiel 1 | Vergleichsbeispiel 2 | Vergleichsbeispiel 3 |
|---|---|---|---|---|---|---|
| | Natriumlactat | Natriumsulfat | La Toja | mit Borax | mit Borax | ohne Borax |
| | | | | | | |
| Edenor L2SM | 32,36 | 32,36 | 32,36 | 32,36 | 32,36 | 32,36 |
| Coconut Oil (refined) | 8,81 | 8,81 | 8,81 | 8,81 | 8,81 | 8,81 |
| Kaliumhydroxid | 7,81 | 7,81 | 7,81 | 7,81 | 7,81 | 7,81 |
| Natriumhydroxid | 1,11 | 1,11 | 1,11 | 1,11 | 1,11 | 1,11 |
| Texapon CS | 2,0 | 2,0 | 2,0 | 2,0 | 2,0 | 2,0 |
| Glycerin | 8 | 8 | 8 | 8 | 8 | 8 |
| Borax | - | - | - | 0,5 | 0,5 | - |
| Natriumlactat | 0,2/0,6/ 0,9/1,2 | - | - | - | - | - |
| Natriumsulfat | - | 0,5/1,0 | - | - | - | - |
| Natriumchlorid (als Mineralsalzmischung aus natürlichem Mineralwasser La Toja, Spanien) | 0,25 | 0,25 | 0,5 | 0,25 | 0,01 | 0,25 |
| Ajidew NL-50 | - | - | - | - | 0,5 | - |
| Parfum | 1,0 | 1,0 | 1,0 | 1,0 | 1,0 | 1,0 |
| Wasser | ad 100 | ad 100 | ad 100 | ad 100 | ad 100 | ad 100 |
| | | | | | | |
| pH | | | | 10-10,2 | 10 - 10,2 | |
| Viskosität* | | | | 120.000 - 200.000 | 120.000 - 200.000 | |
| Gehalt an freien Fettsäuren (als Stearinsäure) | | | | 1,5-2,8 | 1,5 - 2,8 | |

| | | | | | | |
|---|---|---|---|---|---|---|
| * gemessen 5 Tage nach Herstellung, bei 20°C Umgebungs- und Probentemperatur mit einem Brookfield Rotationsviskosimeter Modell RVT, mit Helipath, Spindel TD, 5 Umdrehungen pro Minute | | | | | | |

Die Mengenangaben zu Glycerin beziehen sich immer auf die Menge an zugefügtem Glycerin (86 Gew.-%iges Handelsprodukt), wobei das Glycerin, das durch Verseifung des raffinierten Kokosöls entsteht, nicht berücksichtigt ist.

Die Standardcremes mit Borax (Vergleichsbeispiele 1 und 2) ließen sich gut verarbeiten, entwickelten einen schönen Perlglanz innerhalb von 4 - 5 Tagen nach der Herstellung und wiesen eine angenehme Textur auf. Sie ließen sich leicht aus einer Tube entnehmen.

Die Creme ohne Borax (Vergleichsbeispiel 3) ließ sich ab dem zweiten Verseifungsschritt kaum noch verarbeiten, da sich eine halbfeste Masse bildete, die nicht zu homogenisieren war. Der volle Perlglanz entwickelte sich erst 1 Monat nach der Herstellung. Die Textur der Creme war unzureichend und wies Klumpen auf. Die Creme ließ sich schlecht auf der Haut verteilen.

Die nicht erfindungsgemäße Creme 1a (mit 0,45 Gew.-% Natriumchlorid und Natriumlactat) wies bei der Herstellung eine zu hohe Viskosität auf und konnte nicht ausreichend homogenisiert werden.

Die erfindungsgemäßen Cremes 1b, 1c und 1d (mit 0,6 bzw. 0,9 bzw. 1,2 Gew.-% Natriumlactat) wiesen bei der Herstellung eine Viskosität auf, die über denen von Vergleichsbeispiel 1 bzw. 2 lag. Beim Abkühlen stellte sich aber eine brauchbare Viskosität ein, und das Produkt konnte vollständig homogenisiert werden. Es konnte eine nahezu vollständige Homogenisierung erzielt werden. Die erfindungsgemäßen Cremes 1b, 1c und 1d entwickelten einen schönen Perlglanz innerhalb von 4 - 5 Tagen nach der Herstellung und wiesen eine angenehme Textur auf. Sie ließen sich leicht aus einer Tube entnehmen und auf der Haut verteilen. Die Produktqualität war gut reproduzierbar.

Die erfindungsgemäßen Cremes 2a, 2b und 3 wiesen bei der Herstellung eine Viskosität auf, die zwischen der von Vergleichsbeispiel 3 und denen von Vergleichsbeispiel 1 bzw. 2 lagen. Es konnte eine nahezu vollständige Homogenisierung erzielt werden. Die erfindungsgemäßen Cremes 2a, 2b und 3 entwickelten einen schönen Perlglanz innerhalb von 4 - 5 Tagen nach der Herstellung und wiesen eine angenehme Textur auf. Sie ließen sich leicht aus einer Tube entnehmen.

Weitere erfindungsgemäße Rezepturbeispiele

| | 4 | 5 | 6 | 7 | 8 | 9 | 10 | 11 |
|---|---|---|---|---|---|---|---|---|
| | | | | | | | | |
| Edenor L2SM | 32,36 | 32,36 | 32,36 | 32,36 | 32,36 | 32,36 | 32,36 | 32,36 |
| Coconut Oil (refined) | 8,81 | 8,81 | 8,81 | 8,81 | 8,81 | 8,81 | 8,81 | 8,81 |
| Kaliumhydroxid | 7,81 | 7,81 | 7,81 | 7,81 | 7,81 | 7,81 | 7,81 | 7,81 |
| Natriumhydroxid | 1,11 | 1,11 | 1,11 | 1,11 | 1,11 | 1,11 | 1,11 | 1,11 |
| Texapon CS | 2,0 | 2,0 | 2,0 | 2,0 | 2,0 | 2,0 | 2,0 | 2,0 |
| Glycerin | 8 | 8 | 8 | 8 | 8 | 8 | 8 | 8 |
| Natriumlactat | 1,2 | 0,9 | 1,2 | 0,9 | 0,9 | 1,3 | 0,9 | 1,3 |
| Ajidew NL-50 | - | - | - | - | - | 0,5 | - | 0,5 |
| Allantoin | 0,5 | 0,5 | - | - | - | - | - | - |
| Bisabolol | - | - | 0,5 | 0,5 | - | - | - | - |
| Panthenol | - | - | - | - | 0,3 | - | 0,3 | - |
| Dow Corning Surfactant 190 | - | - | 0,5 | - | - | - | - | 1,0 |
| Polyox WSR 301 | - | - | - | 0,08 | | 0,06 | - | - |
| Dow Corning 2-1184 | 1,0 | - | - | - | - | - | - | 1,0 |
| Fucogel | - | - | - | - | 0,5 | - | - | 1,0 |
| Aloe vera | | 0,5 | | | | | 0,5 | 0,5 |
| Dermosoft Octiol | - | - | 1,0 | 1,0 | - | 1,0 | - | - |
| Methylparaben | 1,0 | 1,0 | 1,0 | 1,0 | - | - | - | - |
| Propylparaben | 1,0 | 1,0 | 1,0 | 1,0 | - | - | - | - |
| Frescolat MGA | 0,5 | 0,5 | 0,5 | - | - | - | - | - |
| Parfum | 1,0 | 1,0 | 1,0 | 1,0 | 1,0 | 1,0 | 1,0 | 1,0 |
| Menthol | - | 0,5 | 0,5 | - | 0,5 | - | 0,5 | - |
| Wasser | ad 100 | ad 100 | ad 100 | ad 100 | ad 100 | ad 100 | ad 100 | ad 100 |

Liste der verwendeten Rohstoffe

| Handelsname | INCl-Bezeichnung | Hersteller |
|---|---|---|
| Dow Corning 2-1184 | Dimethicone, Trisiloxane | Dow Corning |
| Edenor L2SM | Palmitinsäure (40-55 %) und Stearinsäure (40- 55 %) | Cognis |
| Frescolat MGA | Menthone glycerine acetal | Symrise |
| Fucogel | Biosaccharide Gum-1 | Solabia |
| Dermosoft Octiol | 1,2-Octandiol | Dr. Straetmans |
| Polyox WSR 301 | PEG-90 M | Amerchol |
| Texapon CS | Water, Sodium Lauryl Sulfate, Sodium Myristyl Sulfate, Sodium Cetyl Sulfate, Sodium Stearyl Sulfate, Laureth-10 | Cognis |

## Patentansprüche

1. Perlglänzende kosmetische Zusammensetzung, die als Rasiercreme verwendet werden kann, enthaltend
a) Wasser,
b) mindestens eine Monocarbonsäure mit 8 - 24 Kohlenstoffatomen, ausgewählt aus linearen, verzweigten, gesättigten und ungesättigten Monocarbonsäuren mit 8 - 24 Kohlenstoffatomen,
c) mindestens ein Neutralisierungsmittel für b),
d) mindestens einen Elektrolyten, ausgewählt aus Natriumchlorid, Natriumsulfat, Natriumlactat, Natriumglycolat, Natriumcitrat, Kaliumchlorid, Kaliumsulfat, Magnesiumchlorid, Calciumchlorid sowie Mischungen hiervon, in einer Gesamtmenge von 0,5 - 10 Gew.-%, bezogen auf das Gewicht der gesamten Zusammensetzung.

2. Zusammensetzung gemäß Anspruch 1, **dadurch gekennzeichnet, dass** die Gesamtmenge an Monocarbonsäuren mit 8 - 24 Kohlenstoffatomen b) 25 - 50 Gew.-%, bezogen auf das Gewicht der gesamten Zusammensetzung, beträgt.

3. Zusammensetzung gemäß Anspruch 1 oder 2, **dadurch gekennzeichnet, dass** die Gesamtmenge an nicht-neutralisierter Monocarbonsäure/nicht-neutralisierten Monocarbonsäuren mit 8 - 24 Kohlenstoffatomen b) 0,5 - 5 Gew.-%, bevorzugt 1 - 4 Gew.-%, jeweils bezogen auf das Gewicht der gesamten Zusammensetzung, beträgt.

4. Zusammensetzung gemäß einem der vorstehenden Ansprüche, **dadurch gekennzeichnet, dass** die Gesamtmenge an ungesättigter Monocarbonsäure/ungesättigten Monocarbonsäuren b) mit 8 - 24 Kohlenstoffatomen 0,01 - 20 Gew.-%, bevorzugt 0,3 - 2 Gew.-%, jeweils bezogen auf das Gewicht der gesamten Zusammensetzung, beträgt.

5. Zusammensetzung gemäß einem der vorstehenden Ansprüche, **dadurch gekennzeichnet, dass** der Anteil an Laurinsäure 2 - 10 Gew.-%, bevorzugt 3 - 6 Gew.-%, jeweils bezogen auf das Gewicht der gesamten Zusammensetzung, beträgt.

6. Zusammensetzung gemäß einem der vorstehenden Ansprüche, **dadurch gekennzeichnet, dass** die Gesamtmenge an Stearinsäure und/oder Palmitinsäure 20 - 40 Gew.-%, bevorzugt 25 - 35 Gew.-%, jeweils bezogen auf das Gewicht der gesamten Zusammensetzung, beträgt.

7. Zusammensetzung gemäß einem der vorstehenden Ansprüche, **dadurch gekennzeichnet, dass** die mindestens eine Monocarbonsäure b) mit 8 - 24 Kohlenstoffatomen in situ gebildet wird aus mindestens einem Glyceryltriester entsprechender Monocarbonsäuren, der bei 30 °C flüssig ist, und dem Neutralisierungsmittel für b).

8. Zusammensetzung gemäß Anspruch 7, **dadurch gekennzeichnet, dass** der mindestens eine Glyceryltriester ausgewählt ist aus natürlichen Ölen, vorzugsweise aus Avocadoöl, Baumwollsaatöl, Distelöl, Erdnussöl, Kokosöl, Leinöl, Maiskeimöl, Mandelöl, Olivenöl, Palmöl, Palmkernöl, Pfirsichkernöl, Rapsöl, Rizinusöl, Sesamöl, Sojaöl, Sonnenblumenöl und Weizenkeimöl.

9. Zusammensetzung gemäß einem der vorstehenden Ansprüche, **dadurch gekennzeichnet, dass** das Neutralisierungsmittel c) ausgewählt ist aus Kaliumhydroxid, Triethanolamin, Mischungen von Kaliumhydroxid und Natriumhydroxid, Mischungen von Triethanolamin und Natriumhydroxid und Mischungen von Kaliumhydroxid, Triethanolamin und Natriumhydroxid.

10. Zusammensetzung gemäß Anspruch 9, **dadurch gekennzeichnet, dass** die Neutralisierungsmittelmischungen ausgewählt sind aus Mischungen, in denen Kaliumhydroxid im molaren Überschuss zu Natriumhydroxid vorliegt, Mischungen, in denen Triethanolamin im molaren Überschuss zu Natriumhydroxid vorliegt, und Mischungen, in denen die Gesamtmenge an Kaliumhydroxid und Triethanolamin im molaren Überschuss zu Natriumhydroxid vorliegt.

11. Zusammensetzung gemäß einem der vorstehenden Ansprüche, **dadurch gekennzeichnet, dass** die Gesamtmenge an Wasser 30 - 60 Gew.-%, bevorzugt 35 - 50 Gew.-%, jeweils bezogen auf das Gewicht der gesamten Zusammensetzung, beträgt.

12. Zusammensetzung gemäß einem der vorstehenden Ansprüche, **dadurch gekennzeichnet, dass** der mindestens eine Elektrolyt d) in einer Gesamtmenge von 0,6 - 5 Gew.-%, bevorzugt 0,9 - 2,0 Gew.-%, außerordentlich bevorzugt 1,2 - 1,8 Gew.-%, jeweils bezogen auf das Gewicht der gesamten Zusammensetzung, vorliegt.

13. Zusammensetzung gemäß einem der vorstehenden Ansprüche, **dadurch gekennzeichnet, dass** weder Borsäure noch ein Salz der Borsäure noch ein Derivat der Borsäure enthalten ist.

14. Zusammensetzung gemäß einem der vorstehenden Ansprüche, **dadurch gekennzeichnet, dass** maximal 0,2 Gew.-%, bevorzugt maximal 0,1 Gew.-%, besonders bevorzugt 0 Gew.-%, jeweils bezogen auf das Gewicht der gesamten Zusammensetzung, Ethylenglycolmono- und/oder -distearat und/oder Diethylenglycolmono- und/ oder -distearat und/oder Fettsäuremonoethanolamid enthalten ist/sind.

15. Zusammensetzung gemäß einem der vorstehenden Ansprüche, **dadurch gekennzeichnet, dass** mindestens ein wasserlösliches mehrwertiges C₂ - C₉-Alkanol mit 2 - 6 Hydroxylgruppen und/oder mindestens ein wasserlösliches Polyethylenglycol, bestehend aus 3 - 20 Ethylenoxid-Einheiten, sowie Mischungen hiervon, enthalten ist.

16. Zusammensetzung gemäß Anspruch 15, **dadurch gekennzeichnet, dass** mindestens ein wasserlösliches mehrwertiges C₂ - C₉-Alkanol mit 2 - 6 Hydroxylgruppen und/oder mindestens ein wasserlösliches Polyethylenglycol, bestehend aus 3 - 20 Ethylenoxid-Einheiten, sowie Mischungen hiervon, in einer Gesamtmenge von 2 - 20 Gew.-%, bevorzugt 5 - 15 Gew.-%, besonders bevorzugt 7 - 12 Gew.-%, jeweils bezogen auf das Gewicht der gesamten Zusammensetzung, enthalten ist.

17. Zusammensetzung gemäß einem der vorstehenden Ansprüche, **dadurch gekennzeichnet, dass** mindestens ein weiteres anionisches Tensid enthalten ist.

18. Zusammensetzung gemäß Anspruch 17, **dadurch gekennzeichnet, dass** das mindestens eine weitere anionische Tensid ausgewählt ist aus Alkylsulfaten mit 8 - 18 C-Atomen in der Alkylgruppe, Alkylpolyglycolethersulfaten und Ethercarbonsäuren, jeweils mit 8 - 18 C-Atomen in der Alkylgruppe und 1 - 12 Glycolethergruppen im Molekül, Sulfobernsteinsäuremono- und -dialkylestern mit 8 - 18 C-Atomen in der Alkylgruppe, Sulfobernsteinsäuremonoalkylpolyoxyethylestern mit 8 - 18 C-Atomen in der Alkylgruppe und 1 - 6 Glycolethergruppen im Molekül, Monoglyceridsulfaten, Alkyl- und Alkenylphosphaten, Alkyl- und Alkenyletherphosphaten sowie Eiweißfettsäurekondensaten, jeweils mit 8 - 18 C-Atomen in der Alk(en)ylgruppe beziehungsweise in der Acylgruppe und gegebenenfalls 1 - 10 Glycolethergruppen im Molekül.

19. Zusammensetzung gemäß Anspruch 17 oder 18, **dadurch gekennzeichnet, dass** das mindestens eine weitere anionische Tensid in einer Gesamtmenge von 0,1 - 5 Gew.-%, bevorzugt 0,5 - 2 Gew.-%, besonders bevorzugt 1 - 1,5 Gew.-%, jeweils bezogen auf das Gewicht der gesamten Zusammensetzung, enthalten ist.

20. Zusammensetzung gemäß einem der vorstehenden Ansprüche, **dadurch gekennzeichnet, dass** mindestens ein nichtionisches Tensid enthalten ist.

21. Zusammensetzung gemäß Anspruch 20, **dadurch gekennzeichnet, dass** das mindestens eine nichtionische Tensid ausgewählt ist aus Alk(en)yloligoglycosiden mit 8 - 18 C-Atomen in der Alk(en)ylgruppe, Fettsäurediethanolamiden und Aminoxiden sowie Mischungen hiervon.

22. Zusammensetzung gemäß Anspruch 20 oder 21, **dadurch gekennzeichnet, dass** das mindestens eine weitere nichtionische Tensid in einer Gesamtmenge von 0,1 - 2 Gew.-%, bevorzugt 0,5 - 1,5 Gew.-%, besonders bevorzugt 0,7 - 1,0 Gew.-%, jeweils bezogen auf das Gewicht der gesamten Zusammensetzung, enthalten ist.

23. Zusammensetzung gemäß einem der vorstehenden Ansprüche, **gekennzeichnet durch** eine Viskosität von 80.000 - 300.000 mPas, bevorzugt 120.000 - 200.000 mPas, jeweils gemessen 5 Tage nach Herstellung, bei 20°C Umgebungs- und Probentemperatur mit einem Brookfield Rotationsviskosimeter Modell RVT, mit Helipath, Spindel TD, 5 Umdrehungen pro Minute.

24. Zusammensetzung gemäß einem der vorstehenden Ansprüche, **dadurch gekennzeichnet, dass** mindestens ein Polyethylenglycol mit einem mittleren Molekulargewicht von mindestens 300.000 Dalton und der allgemeinen Strukturformel H-(OCH₂CH₂)ₙ-OH in einer Menge von 0,001 - 0,2 Gew.-%, bezogen auf die gesamte Zusammensetzung, enthalten ist.

25. Zusammensetzung gemäß einem der vorstehenden Ansprüche, **dadurch gekennzeichnet, dass** mindestens ein Polyalkylenglycol-modifiziertes Silicon enthalten ist.

26. Zusammensetzung gemäß einem der vorstehenden Ansprüche, **dadurch gekennzeichnet, dass** mindestens ein Öl und/oder mindestens ein Fettstoff ohne Estergruppe im Molekül enthalten ist.

27. Zusammensetzung gemäß einem der vorstehenden Ansprüche, **dadurch gekennzeichnet, dass** mindestens eine Kieselsäure enthalten ist.

28. Zusammensetzung gemäß einem der vorstehenden Ansprüche, **dadurch gekennzeichnet, dass** mindestens ein hautkühlender Wirkstoff enthalten ist.

29. Zusammensetzung gemäß einem der vorstehenden Ansprüche, **dadurch gekennzeichnet, dass** mindestens ein hautberuhigender Wirkstoff enthalten ist.

30. Zusammensetzung gemäß einem der vorstehenden Ansprüche, **dadurch gekennzeichnet, dass** mindestens ein hautfeuchthaltender Wirkstoff enthalten ist.

31. Nicht-therapeutische Verwendung einer Zusammensetzung gemäß einem der Ansprüche 1 - 30 als Rasierhilfsmittel oder als Reinigungsmittel für die Haut und/oder das Haar.

32. Verwendung einer Zusammensetzung gemäß einem der Ansprüche 1 - 30 zur Herstellung einer topischen Zusammensetzung zur therapeutischen Behandlung der Haut, der Schleimhäute und/oder der Kopfhaut.

33. Nicht-therapeutische Verwendung einer kosmetischen Zusammensetzung gemäß einem der Ansprüche 1 - 30 zur Befeuchtung und/oder als Moisturizer und/oder zur Erfrischung und/oder als anti-irritatives Rasierhilfsmittel oder als anti-irritatives Reinigungsmittel für die Haut und/oder das Haar.

34. Nicht-therapeutisches, kosmetisches Verfahren zur Rasur und/oder zur Reinigung der Haut und/oder des Haars, bei dem eine kosmetische Zusammensetzung gemäß einem der Ansprüche 1 - 30 auf die Haut und/oder die Haare aufgetragen, gewünschtenfalls eine Rasur erfolgt und anschließend gewünschtenfalls abgespült und/oder abgewischt wird.
